# EUROPEAN PATENT APPLICATION

(11) **EP 2 002 848 A2**
(43) Date of publication of application: **17.12.2008**
(21) Application number: 07739338.7
(22) Date of filing: 22.03.2007
(51) Int. Cl.: A61L 9/01, A61K 8/368, A61K 8/49, A61Q 13/00, C11D 3/20, C11D 3/386, C11D 3/50, C11D 17/06

(54) **LOCK-IN TYPE POWDER**

(30) Priority: 22.03.2006 JP 2006079796
(71) Applicant: Takasago International Corporation, Tokyo 144-8721 (JP)
(72) Inventor: SHIROYAMA, Kenichiro, (JP); HIRAMOTO, Tadahiro, (JP); KUMAMOTO, Hiroyasu, (JP)
(74) Representative: Jones, Helen M.M.
(86) International application number: PCT/JP2007/055895
(87) International publication number: WO 2007/119492

(57) **Abstract**

The present invention relates to a locked-in type powder comprising polyphenols, the locked-in type powder which is excellent in long-term stability, substantially does not show decrease in color tone and discoloration even after long-term storage, also does not result in change in odor quality and decrease in deodorizing and antioxidizing effects, and is capable of retaining good effects over a long period of time, and a solid preparation comprising the same.

## Description

### Technical Field

The present invention relates to a locked-in type powder comprising polyphenols, and a solid preparation comprising the same. More specifically, the invention relates to a locked-in type powder comprising polyphenols, the locked-in type powder which is excellent in long-term stability, substantially does not show decrease in color tone and discoloration even after long-term storage, also does not result in change in odor quality and decrease in deodorizing and antioxidizing effects, and is capable of retaining a good effect over a long period of time, and a solid preparation comprising the same. In particular, it relates to a solid preparation in which a deterioration is hardly generated by components such as a basic ingredient such as sodium sulfate or sodium hydrogen carbonate, a surfactant, and an enzyme and the like, which is contained in a bath agent, a detergent, or the like.

### Background Art

In recent years, with diversification of lives, improvement of life level, changes and improvement of attitudes and the like, an attention has been paid to various points around one's life. In accordance therewith, a variety of fragrances or cosmetics such as a powdery bath agent, a tablet bath agent (bath tablet), a bubble bath, a deodorant powder, a powder detergent, a detergent for an automatic dish washer, a powder bleach, a powder pack, a whitening powder, a sliminess remover, a false teeth cleanser, and the like have been commercially available and thus a consumer's satisfactory has been increased.
One of the various fragrances or cosmetics, there is a fragrance or cosmetic in which care is taken to familiar smells such as bad breath and smell of sweat. Most of them are products having a so-called masking effect that makes the smells insensitive, but on the other hand, there has been investigated a product having a so-called deodorizing effect that traps a malodor component, and for example, there has been investigated a development of fragrances or cosmetics in which a deodorizing active ingredient is blended into the fragrances or cosmetics. Most of the above deodorizing ingredients are those contained in a plant extract (Patent Document 1, Patent Document 2, etc.). The deodorizing ingredients are derived from a natural substance and can be used safely and also are excellent in deodorizing effect, but they are not said to provide a satisfactorily sufficient effect.
Accordingly, when the present inventors have investigated to develop a technology exhibiting a more excellent deodorizing function, they have obtained a finding that a deodorant composition in which polyphenols, which are known to be present in a plant, and an alkali ingredient are coexisted is excellent in deodorizing effect and also has a characteristic that the composition can be used safely like the above deodorant, and thus they have started a study that the finding is applied.
With attention to a point that an alkali ingredient is usually blended into a fragrance or cosmetic such as a detergent and a bath agent, when it is attempted to blend polyphenols into the above fragrance or cosmetic, there has been obtainable a fragrance or cosmetic to which an excellent deodorizing function is enhanced.
However, with regard to the above fragrance or cosmetic comprising the polyphenols, there have been found new problems that the fragrance or cosmetic itself is colored (discolored) and also odor quality vaporized from the fragrance or cosmetic changes after the passage of a long period of time except for an initial stage.
The product in which the coloration and change in odor quality are observed has a large problem that an image as a commercial article is bad for consumers and cosequently a commercial value has been lost although the function of the product itself is by no means problematic. Therefore, it is desired to develop a new technology so that the above deodorization technology excellent in deodorizing effect can be put into practical use for fragrances or cosmetics.

On the other hand, a locked-in type powder comprising an flavor ingredient locked therein is, for example, produced by a method in which a carbohydrate such as a glucide is heated with a small amount of water, if necessary, to obtain an amorphous matrix material as a melt form, then a usually liquid flavor ingredient is mechanically dispersed and locked-in the amorphous matrix material, and after it is solidified by cooling or the like as it is, pulverization is conducted, or the above locked-in one is extrued using a single-screw or twinscrew extruder into a cooling material such as a cooling medium through a die to solidify the one and then it is cut or pulverized, which is already known. The above locked-in type powder has been hitherto used for enhancing a flavor to various foods and, particularly, is widely used in the production of a candy, a chewing gum, and the like.

As the flavor ingredient-locked-in locked-in type powders or processes for producing the same, which have hitherto proposed, the following technologies may be mentioned, for example.
(1) A method of dispersing a flavoring oil (flavor ingredient) comprising an antioxidant and a dispersant in a heated corn syrup, extruding and molding the resulting dispersion, and then crushing it (see Patent Document 3).
(2) A method of heating a mixture comprising sugars such as sucrose, a starch hydrolyzate, and an emulsifier, blending a flavor ingredient thereinto, extruding the resulting one into a cold solvent, molding it, and then pulverizing the resulting molded one (see Patent Documents 4 to 7).
(3) A method of immobilizing an essential oil or the like with an aqueous sorbitol solution or a molten solution thereof (see Patent Document 8).

Patent Document 1: JP-A-9-290014
Patent Document 2: JP-A-9-290026
Patent Document 3: JP-B-34-5600
Patent Document 4: JP-A-49-62677
Patent Document 5: JP-A-52-94452
Patent Document 6: JP-A-61-12248
Patent Document 7: JP-T-61-502656
Patent Document 8: JP-A-49-132251
Non-Patent Document 1: "PRINCIPLES OF FOOD CHEMISTRY" Second Edition written by Jhon M. deMAN, published by Van Nostrand Reinhold (New York), 1990, pages 167-168

These flavor ingredient-locked-in locked-in type powders have hitherto been blended into or added to foods and the like and have used for preventing vaporization of the flavor ingredient, a flavor, and the like in the capsules during storage or commercialization of the foods, and used for releasing the flavor ingredient and the like in the capsules into the mouth when the foods and the like are charged into the mouth of consumers so that the consumers recognize the taste or odor of the foods and the like with sufficient satisfaction. Therefore, it has not been considered to use the locked-in type powders for the purposes other than the release of the flavor ingredient, flavor and the like into the mouth and, needless to say, there has not been considered a use that the above locked-in type powders are blended into fragrances or cosmetics whose type of usage is different from that of such foods or drinks and the locked-in compound is released when used. To the applicant's knowledge, fragrances or cosmetics blended into the above locked-in type powders are hitherto not present in the market.

### Disclosure of the Invention

An object of the invention is to provide a solid preparation which is excellent in storage stability, does not show discoloration, decrease in color tone, and change in odor quality even after long-term storage, and also retains a deodorizing function. Furthermore, it is to provide a solid preparation effective for production of fragrances or cosmetics without discoloration, decrease in color tone and change in odor quality, and decrease in deodorizing and antioxidizing functions even after the passage of a standard period of time from the start to the end of consumer's use of the products after the fragrance or cosmetic is shipped from a factory. Furthermore, it is to provide a locked-in type powder having a deodorizing function to be incorporated into the above solid preparation for fragrance or cosmetic blending. The solid preparation in the invention means a solid preparation having a certain shape, such as a tablet, a pill, a capsule, a granule, a fine granule, or a powder. Moreover, the solid preparation of the invention means a fragrance or cosmetic or a raw material for producing a fragrance or cosmetic. A fragrance or cosmetic can be obtained by applying a conventional means to a solid preparation as a raw material for producing the fragrance or cosmetic.
Also, an object of the invention is to provide a solid preparation for fragrances or cosmetics, which does not show discoloration, decrease in color tone, and change in odor quality by an additive, e.g., a basic ingredient such as sodium sulfate, sodium carbonate, sodium phosphate, or sodium hydrogen carbonate, which is frequently used as a base ingredient of a fragrance or cosmetic such as a bath agent or a detergent, or an enzyme, and which also retains a deodorizing function.

The present inventors have extensively investigated for solving the above problems but satisfactory results could not be easily obtained. Under such a situation, when a locked-in type powder wherein polyphenols is locked in sugars (hereinafter sometimes referred to as locked-in type powder) is prepared as a result of hard effort and the locked-in type powder is incorporated into a fragrance or cosmetic, then they have found a fact that discoloration, decrease in color tone, and change in odor quality of the solid preparation for the fragrance or cosmetic are surprisingly not observed even after the fragrance or cosmetic is stored for a long period of time. Furthermore, they have also recognized that the solid preparation for the fragrance or cosmetic after long-term storage retains deodorizing and antioxidizing functions. Based on these findings, the inventors have further studied intensively and finally accomplished the invention.

Namely, the invention relates to the following (1) to (10).
(1) A locked-in type powder comprising a polyphenols.
(2) The locked-in type powder according to (1), wherein a content of the polyphenols is 0.2 to 20% by weight based on the total weight of the locked-in type powder.
(3) The locked-in type powder according to (1) or (2), wherein the polyphenols is locked in a matrix material.
(4) The locked-in type powder according to any one of (1) to (3), wherein the polyphenols is a polyphenol having a hydroquinone or an o-diphenol structure.
(5) The locked-in type powder according to any one of (1) to (4), which further comprises at leaset one of a flavor and a fragrance.
(6) A solid preparation comprising the locked-in type powder according to any one of (1) to (5).
(7) The solid preparation according to (6), wherein the solid preparation is a fragrance or cosmetic.
(8) The solid preparation according to (7), wherein the fragrance or cosmetic is a bath agent or a detergent.
(9) The solid preparation according to any one of (6) to (8), which further comprises a basic ingredient.
(10) The solid preparation according to any one of (6) to (8), which further comprises an enzyme.
Namely, the invention according to the above (1) is a locked-in type powder comprising a polyphenols. It is also a locked-in type powder for fragrance or cosmetic blending, in which a polyphenols are locked. Furthermore, the invention according to the above (1) may be said to be a locked-in type powder, wherein a polyphenols are locked in sugars or a sugar matrix.
Furthermore, the invention according to the above (1) is also a locked-in type powder comprising a polyphenols. Moreover, the invention according to the above (1) is also a locked-in type powder, wherein a polyphenols is contained in sugars or a sugar matrix. The sugars used here are not particularly limited so far as it is sugars capable of producing the locked-in type powder.

### Best Mode for Carrying Out the Invention

The following will describe the invention further in detail.
The polyphenols (hereinafter sometimes referred to as a polyphenol) as an essential component in the invention will be described.
The polyphenol to be used in the invention is not particularly limited so far as it is a polyphenol capable of attaining the intended purpose. Specifically, the polyphenol to be used in the invention means a compound in which two or more hydrogen atoms on one identical benzene ring are substituted with hydroxyl groups, and glycosides thereof are also included as the polyphenol. Among them, a polyphenol having a hydroquinone and an o-diphenol structure are preferred. The o-diphenol structure means such a structure that hydroxyl groups are directly substituted on the benzene ring and the hydroxyl groups are adjacent to each other.

Specific examples of the polyphenol include apigenin, an apigenin glycoside, acacetin, isorhamnetin, an isorhamnetin glycoside, isoquercitrin, epicatechin, epicatechin gallate, epigallocatechin, epigallocatechin gallate, aesculetin, an ethyl protocatechuate salt, ellagic acid, catechol, gamma-acid, catechin, gardenin, gallocatechin, caffeic acid, a caffeic ester, chlorogenic acid, kaempferol, a kaempferol glycoside, quercetin, a quercetin glycoside, quercetagenin, genistin, a genistin glycoside, gossypetin, a gossypetin glycoside, gossypol, 4-dihydroxyanthraquinone, 1,4-dihydroxynaphthalene, cyanidin, a cyanidin glycoside, sinensetin, diosmetin, a diosmetin glycoside, 3,4'-diphenyldiol, sinapic acid, stearyl-β-(3,5-di-t-butyl-4-hydroxyphenyl)propionate, spinacetin, tangeritin, taxifolin, tannic acid, daphnetin, tyrosine, delphinidin, a delphinidin glycoside, theaflavine, theaflavine monogallate, theaflavine bisgallate, tricetinidin, dopa, dopamine, naringenin, naringin, nordihydroguairetic acid, noradrenaline, hydroquinone, vanillin, patchouletin, herbacetin, vanillyl alcohol, vanitrope, vanillin propylene glycol acetal, vanillic acid, bis(4-hydroxyphenyl)sulfonic acid, bisphenol A, pyrocatechol, vitexin, 4,4'-biphenyldiol, 4-t-butylcatechol, 2-t-butylhydroquinone, protocatechuic acid, phloroglucinol, a phenolic resin, procyanidin, prodelphinidin, phloretin, a phloretin glycoside, fisetin, folin, fervasetin, fraxetin, phloridzin, paeonidin, a paeonidin glycoside, pelargonidin, a pelagugonidin glycoside, petunidin, a petunidin glycoside, hesperetin, hesperidin, gallic acid, a gallic ester (lauryl gallate, propyl gallate, butyl gallate), manjiferin, malvidin, a malvidin glycoside, myricetin, a myricetin glycoside, 2,2'-methylenebis(4-methyl-6-t-butylphenol), 2,2'-methylenebis(4-ethyl-6-t-butylphenol), 2,2'-methylenebis(4-methyl-6-t-butylphenol), 2,2'-methylenebis(4-ethyl-6-t-butylphenol), methyl atrarate, 4-methylcatechol, 5-methylcatechol, 4-methoxycatechol, 5-methoxycatechol, methylcatechol-4-carboxylic acid, 2-methylresorcinol, 5-methylresorcinol, morin, limocitrin, a limocitrin glycoside, liniocitrol, luteolin, a luteolin glycoside, luteolinidin, a luteolinidin glycoside, rutin, resorcin, resveratrol, resorcinol, leukocyanidin, leukodelphinidin, and the like.

Among these polyphenols, particularly preferred are flavonoids such as quercetin, epicatechin, and epigallocatechin and glycosides thereof; polyphenols having an o-diphenol structure such as gallic acid, a gallic ester, chlorogenic acid, caffeic acid, a caffeic acid ester, tannic acid, pyrocatechol, nordihydroguairetic acid, L-dopa, 4-methylcatechol, 5-methylcatechol, 4-methoxycatechol, and 5-methoxycatechol; and hydroquinone.
These polyphenols may be used each alone or as a mixture of two or more of them.
Moreover, the above polyphenol can be prepared by known methods, but a commercially available product may be purchased. Moreover, it may be prepared by synthesis. Furthermore, polyphenol fractions of a high concentration prepared from plants can be employed.

In the invention, instead of the polyphenol, a polyphenol-containing plant extract can also be used. The plant extract in this case comprises a polyphenol and a plant extract containing substantially no amino acid can be also adopted. As the plant extract, one prepared by a known method may be used or a commercially available product may be used.
Examples of the plant extract include extracts obtained from aloe, anise seeds, elder, eleutherococcus, psyllium, orange flower, allspice, oregano, valerian, chamomile, capsicum pepper, cardamon, cassia, garlic, caraway seeds, clove, cumin seeds, kola, coriander seeds, oak apple, saffron, zanthoxylum, juniper berry, cinnamon, ginger, star anise, St. Johns wart, celery seed, savory, sesame, pieplant, tarragon, turmeric, thistle, dill seed, nutmeg, nettle, hibiscus, hamamelis, birch, basil, bitter orange, fennel, primrose, fenugreek, verbena, bay laurel, hop, boldo, horseradish, poppy seed, gallnut, marigold, marrow, marjoram, mustard, Millefeuille, mint leaves, melissa, mace, lindane, gentiana, rosehip, rosemary, Rosmarinus officinalis, sunflower seeds, grape pericarp, apple, carrot leaves, banana, strawberry, apricot, peach, plum, pineapple, Nashi pear, persimmon, cherry, papaya, mango, avocado, melon, loquat, fig, kiwi, prune, blueberry, black berry, raspberry, cranberry, coffee beans, cacao beans, grape seeds, grape fruits seeds, pecan nut, cashew nut, chestnut, coconut, peanut, walnut, green tea leaves, black tea leaves, oolong tea leaves, tobacco, perilla leaves, garden thyme, sage, lavender, spearmint, peppermint, spotted thistle, hyssop, sweet basil, marigold, dandelion, artichoke, Matricaria chamomille, agrimonia eupatorina, licorice, anise, yarrow, eucalyptus, wormwood, balm, Angelica pubescens, fenugreek, Capsicum annuum var. angulosum, fennel, red pepper, coriander seeds, caraway seeds, fennel seeds, ginger, horseradish, Origanum majorana, Origanum valgare, mustard, parsley, pepper, savory, tarragon, queen lily, wasabi, dill seeds, citrus fruits, and the like. These plant extracts may be used in combination.
In the invention, since the content of the polyphenols varies depending on the kind of the solid preparation to be blended, the ingredient to be incorporated, etc., it cannot be categorically defined, but the polyphenols content may be 0.2 to 20% by weight, preferably 1 to 15% by weight based on the total amount of the locked-in type powder. The blending amount of the plant extract may be the same as above.

When the matrix material for the locked-in type powder of the invention is specifically depicted, examples thereof include monosaccharides, disaccharides, polysaccharides, sugar alcohols, polyols, saccharide derivatives, processed starches, modified starches, and gums. More specifically, they include, for example, sucrose, glucose, lactose, levurose, fructose, maltose, glucopyranosyl mannitol, glucopyranosyl sorbitol, ribose, dextrose, isomalt, sorbitol, mannitol, xylol, lactitol, maltitol, pentatol, arabinose, pentose, xylose, galactose, starch, hydrogenated starch hydrolyzates, maltodexctrin, agar, carrageenan, and polydextrose, as well as derivatives and mixtures thereof.

The above matrix materials are all preferable as a matrix material for the locked-in type powders to be used in the invention. Among them, one preferable example is a carbohydrate mixture comprising a processed starch and a hydrogenated product of sugars in a weight ratio of 1:99 to 30:70 in terms of solid matter. As the hydrogenated product of the sugars, one or more substances selected from xylitol, lactitol, maltitol, isomalt, hydrogenated treated products thereof, and hydrogenated corn syrup are preferred.

The processed starch here is a chemically treated starch and examples thereof include a starch treated with an acid (example: dextrin), a starch treated with an enzyme (example: dextrin), a heated starch (example: α-starch), an oxidized starch, a crosslinked starch, and the other starch derivatives (e.g., see Non-Patent Document 1). As the dextrin, one having a low DE (Dextrose Equivalent = dextro-rotating sugar equivalent) value, particularly one having the value of about 20 or less is preferably used. When the DE value is larger than about 20, stability against water becomes worse. However, when the DE value is less than about 5, there is a fear that viscosity at polyphenol mixing becomes too high. Moreover, the starch derivative is a highly processed starch wherein side chains of the starch are modified with a hydrophilic group or a hydrophobic group. Specifically, there may be mentioned a starch ester such as a starch phosphate ester, a starch ether such as a starch carboxymethyl ether, and a crosslinked starch such as a phosphoric acid-crosslinked starch. These starch derivatives are excellent in stability of the resulting capsules against water during storage as compared with the other processed starches and thus are preferably used. Furthermore, as the other element enhancing water stability of the capsules, use of a processed starch having a large average molecular weight and a branched structure may be mentioned. As the processed starch having a branched structure, there may be mentioned one wherein a starch comprising amylopectin in a high ratio is processed. In particular, one wherein a starch comprising amylopectin in an amount of about 80% by weight or more is processed is preferably used.

Specific examples of such processed starches include processed starches derived from an amioca starch, a tapioca starch, a waxy maize and the like. Of these, a processed starch derived from a waxy maize is particularly preferred. As the processed starch most preferably used, there may be mentioned a starch derivative which is a processed starch derived from a waxy maize and has a bulky steric structure having a stronger interaction between side chains of the starch. Specific examples of such processed starches include PURITY GUM 59, PURITY GUM BE, PURITY GUM 1773, N-LOK (all trade names, edible processed starches manufactured by National Starch and Chemical Company), and the like. The use of the above matrix results in no deterioration of the polyphenol by the base material, enables gradual release of the polyphenol over a long period of time at the time of use of fragrances or cosmetics, and can retain deodorizing function and antioxidizing function over a long term.

Moreover, as the other preferable matrix material, there may be mentioned one wherein a modified alpharized starch having a viscosity of 50 cps (mPa·s) or more when measured at 30°C after the 20% aqueous solution is heated at 80°C for 5 minutes (hereinafter referred to as "modified alpharized starch (≥50 cps)") as described in JP-A-2005-194419 is used as at least a part of the matrix material. The modified alpharized starch is obtained by rapidly dehydrating and drying, in a paste state, a paste prepared by swelling or dissolving a modified starch with water or hot water, and it can be dispersed and swollen with water at room temperature to easily reproduce a paste. As the modified starch which is a base of the modified alpharized starch, there may be mentioned various processed starches such as an oxidized starch, a starch derivative (a starch acetate ester, a starch phosphate ester, and various starch ether phosphates, a crosslinked starches or the like).

The above modified alpharized starch (≥50 cps) is hitherto known and the various products are commercially available, so that these commercially available ones may be suitably used. For example, as a commercially available one, "N Creamer 46" manufactured by National Starch and Chemical Company is mentioned. The modified alpharized starch (≥50 cps) may be used together with the other modified alpharized starch. As the other modified alpharized starch used together with the above modified alpharized starch (≥50 cps), for example, there may be mentioned a modified alpharized starch having a viscosity of less than 50 cps (mPa·s) when measured at 30°C after the 20% aqueous solution is heated at 80°C for 5 minutes (hereinafter referred to as "modified alpharized starch (<50 cps)"). The modified alpharized starch (<50 cps) is obtained by rapidly dehydrating and drying, in a paste state, a paste prepared by swelling or dissolving the above processed starches (modified starches) such as a hydrolyzed starch, an oxidized starch, and a starch derivative (a starch acetate ester, a starch phosphate ester, various starch ether phosphates, a crosslinked starch) with water or hot water, and they are hitherto known and various products are commercially available same as in the case of the modified alpharized starch (≥50 cps). For example, as commercially available ones, "PURITY GUM BE", "Capsule", and the like manufactured by National Starch and Chemical Company may be mentioned. The combined use thereof enables gradual release of the polyphenol over a longer period of time at the time of use of fragrances or cosmetics and can retain deodorizing function and antioxidizing function over a long term.

By adjusting a blending ratio of the modified alpharized starch (≥50 cps) and the modified alpharized starch (<50 cps) forming aqueous solutions different in viscosity, a vaporization rate and vaporization pattern of the locked-in type powder can be satisfactorily regulated. Moreover, the deterioration and change with time of the polyphenol by the base material contained in a bath agent or a detergent (e.g., thorium marking sheet sulfate, sodium hydrogen carbonate, or the like) can be prevented to retain the deodorizing function and antioxidizing function for a long period of time. Furthermore, the blending ratio of the modified alpharized starch to the total amount of the matrix material is preferably 50% by mass or more, more preferably 60 to 100% by mass, further preferably 70 to 95% by mass or more based on the matrix base material in view of easiness of production, long-term storability, deodorizing function and antioxidizing function, performance of gradual release, touch at use, and the like of the locked-in type powder. Also, the blending ratio of the modified alpharized starch (≥50 cps) is preferably 15% by mass or more, more preferably 20% by mass or more, further preferably 20 to 80% by mass based on the total amount of the matrix material. Moreover, in the case where the modified alpharized starch (≥50 cps) and the modified alpharized starch (<50 cps) are used in combination, the use ratio is generally preferably 10:90 to 80:20 in a mass ratio, more preferably 20:80 to 70:30 in a mass ratio.

Also, the modified alpharized starch may be used in combination with the other components such as sugars. Examples of the sugars usable in combination include monosaccharides such as glucose, fructose, mannose, galactose, xylose, arabinose, and ribose, disaccharides such as mantose, lactose, and sucrose, dextrins obtained by hydrolysis of starches with an acid or an enzyme, sugar alcohols such as mannitol, sorbitol, and malbitol, and the like. Of these, dextrin is preferred. In the case where the modified alpharized starch (≥50 cps) is used as a matrix material, when the formed locked-in type powder is, for example, used for a powder or a tablet bath agent, there is an advantage that the deodorizing function and antioxidizing function are retained for a long period of time even when it is added into hot water having a relatively high temperature of 38 to 42°C.

In the above, the value of the viscosity of a 20% aqueous solution of the modified alpharized starch is a value measured as follows: 40 g of the modified alpharized starch is added to 160 g of water at a temperature of 20°C to form a 20% aqueous solution, the aqueous solution is heated to 80°C under stirring, kept at 80°C for 5 minutes under stirring, and then cooled to 30°C, and the viscosity of the aqueous solution immediately after the temperature reaches 30°C is measured using a viscometer ("Tokimec Viscmeter BM Type"; Spindle No. 1 30 rpm, Spindle No. 2 30 rpm).

As a flavor and/or fragrance to be locked in the locked-in type powder of the invention, any of the flavors or fragrances may be used with no particular limitation including those hitherto used in solid preparations for fragrances or cosmetics so far as they are usable for imparting an odor to fragrances or cosmetics. The flavors or fragrances to be used may be either synthesis aromachemicals or natural essential oils and may be in any of liquid, paste, or solid states. For example, the flavors (hereinafter sometimes referred to as flavors or fragrances) used in the invention include synthetic aromachemicals such as esters, alcohols, aldehydes, ketones, acetals, phenols, ethers, lactones, furans, hydrocarbons and acids, as well as natural origin aromatic materials.
The fragrance (hereinafter sometimes referred to as flavors or fragrances) used in the invention include hydrocarbons, alcohols, phenols, aldehydes and/or acetals, ketones and/or ketals, ethers, synthetic musks, acids, lactones, esters, halogen-containing compounds, and natural origin aromatic materials.

According to the scent and odor quality required for a fragrance or cosmetic, fragrances having desired odor quality and scent can be used. Examples thereof include a citrus-type fragrance, a fruity-type fragrance, a green-type fragrance, a floral-type fragrance, an aldehyde-type fragrance, a spicy-type fragrance, a woody-type fragrance, a sweet-type fragrance, a mossy-type fragrance, a musky-type fragrance, an amber-type fragrance, an animal-type fragrance, a herbal-type fragrance, a marine-type fragrance, a mint-type fragrance, and the like.

As the flavors and fragrances described above, flavors or fragrances described in "Nihon ni Okeru Syokuhin Kouryou Kagoubutu no Shiyou Jittai Cyousa" (Welfare and Science Research Report in 2000; published from Japan Flavor & Fragrance Materials Association, March, 2001, "Gousei Kouryou, Kagaku to Syouhin Chishiki" (written by Motoichi Indo, published on March 6, 1996, The Chemical Daily Co., Ltd.), "Perfume and Flavor Chemicals (Aroma Chemicals) 1, 2" (Steffen Arctender (1969)) or the like can be used.
These flavors and the fragrances may be used each alone or two or more of them may be used in admixture.
A commercial product may be used for them. Moreover, for the single component products, synthesis products may be used or they may be introduced from natural origin such as plants. Essential oils, resinoids, balsams, absolutes, concretes, tinctures, and the like may be prepared by known methods and then used.

Specific examples of the flavor or fragrance ingredient include hydrocarbon-type aromachemicals such as limonene, pinen, γ-terepinen, and caryophyllene; alcohol-type aromachemicals such as phenyl ethyl alcohol, terepineol, bacdanol, geraniol, nerol, linalool, and cis-3-hexenol; aldehyde-type aromachemicals such as lilial, citral, aldehyde C-8, aldehyde C-9, aldehyde C-11, hexyl cinnamic aldehyde, vanillin, and heliotropin; ketone-type aromachemicals such as ionone, rosephenone, woody flow, damascone, Iso E super; other aromachemical such as musks, eugenol and coumarin; essential oils such as a lemon oil, an orange oil, and a peppermint oil; and essences such as an apple essence and a strawberry essence; and the like. In the invention, one or more of the flavor or fragrance ingredients are contained.

Furthermore, together with the flavors or fragrances, if necessary, additives such as a solvent (ethanol, isopropanol, ethylene glycol, propylene glycol, diethylene glycol, dipropylene glycol, glycerin, etc.), an emulsifier, a solubilizer, a retainer, an odor retainer, a cooling agent, a warming agent, an enhancer, an antioxidant, and a photo-degradation inhibitor can also be used properly (see, e.g., "Syuuchi Kanyou Gijyutsusyu (Kouryou) Daiichibu, Kouryou Ippan" published from Japan Patent Office, on January 29, 1999).

The above matrix material, polyphenols, and additives including at least one of a flavor and a fragrance to be used according to need are made into a locked-in type powder by the following method, for example.
First, polyphenols dispersed in water are blended into a matrix material. On this occasion, flavors or fragrances and the other additives can be added according to need. The amount of the polyphenols to be added may be about 0.2 to 20% by weight, preferably 1 to 15% by weight based on the matrix material. The stability of the resulting powder against water varies depending on the content of water, the polyphenols, the flavor or fragrance, and the other additives, and when the content thereof is too large at molding, the stability becomes worse. Therefore, the amount of the polyphenols to be added is desirably about 20% by weight or less based on the matrix material. When the amount of the polyphenols to be added is too small, the effect is not obtained. Therefore, the blending ratio of the polyphenols to the matrix material is usually about 0.2 to 20% by weight, preferably about 1 to 15% by weight. The amount of the flavor or fragrance to be added varies depending on the solid preparation for fragrance or cosmetic blending and thus cannot be categorically defined but, for example, the amount is usually about 0.2 to 20% by weight based on the matrix material.
The amount of water for dispersing the polyphenols is desirably a minimum amount necessary for homogeneous dispersion of the polyphenols. The amount varies depending on the kind and amount of the polyphenol to be used and the kind and amount of the matrix material to be used but, for example, is preferably about 3 to 40% by weight based on the matrix material. When the amount of water to be used is too large, too much time is required for lowering the water content by later heating, so that the case is not preferred. The heating temperature depends on the composition but is preferably in the range of about 110 to 200°C and the heating time is determined so that the matrix material is melted and the water content is lowered to some extent. Preferably, boiling-down is continued in a boiling-down pot until the water content decreases to about 0.5 to 6% by weight. When the remaining water content is too large at this point, the water content of the resulting powder becomes large and stability against water becomes worse, so that the case is not preferred. As above, a homogeneous mixture of the matrix material and the polyphenols can be obtained.

The homogeneous mixture of the matrix material and the polyphenols can be also obtained even by a method different from the above method. Namely, after water is added according to need, the matrix material is heated to form a melt of the matrix material. In the case where water is added, the amount thereof is desirably a minimum amount necessary for homogeneous dispersion of the matrix material and, for example, is preferably about 3 to 40% by weight. When the amount of water to be added is too large, too much time is required for lowering the water content by later heating, so that the case is not preferred. The heating temperature depends on the composition thereof but is preferably in the range of about 110 to 200°C and is determined so that the matrix material is melted and the water content is lowered to some extent. Preferably, boiling-down is continued in a boiling-down pot until the water content decreases to about 0.5 to 6% by weight. When the remaining water content is too large at this point, the water content of the resulting powder becomes large and stability against water becomes worse, so that the case is not preferred.
Then, the polyphenols are added to the resulting melt. The amount of the polyphenols to be added is the same as above.

The method for mixing the melt of the matrix material and the polyphenols is not particularly limited so far as it is a method capable of obtaining a homogeneous mixture. For example, the melt of the matrix material may be stirred with a device such as a highspeed stirrer (homodisper), the polyphenols or the like may be added thereto, and stirring may be further continued to be homogeneous. On this occasion, when a dispersant or an emulsifier is blended into the polyphenols or the like, it is easy to obtain a homogeneous dispersion of the polyphenols or the like.

The homogeneous mixture obtained by the above method is transferred to an extruder and, after it is tightly closed, it is pressurized to extrude from a die of the extruder. Also, it is possible to use an extruder comprising a withstand pressure vessel fitted with an extrusion plate, wherein heating, mixing, and extrusion can be performed in the same vessel. Namely, when the matrix material, water to be added, polyphenols, flavor or fragrance and the like are each introduced into the extruder using a feeding device comprising a metering pump or the like, and the heating, mixing, and extrusion are performed in the extruder, a series of the steps can be continuously carried out without changing the vessel and production volume per hour can be increased, so that the case is preferred. Particularly, further preferred is one having a structure capable of performing heating and mixing simultaneously with the transfer of a raw material (hereinafter referred to as "barrel").

Furthermore, an extruder comprising a withstand pressure vessel having two or more screws is preferably used. The extruder having two or more screws is excellent in transferring ability and mixing ability as compared with the extruder comprising a single screw and thus enables more stable production. An extruder having two or more screws in the above barrel is particularly preferably used.

On the other hand, the solidification of the matrix mixture is usually performed with a cooled cooling medium. Therefore, the cooling medium is placed in a cooling tank and is suitably cooled to low temperature. As the cooling medium, it is desirable to select one which is not harmful to human, does not dissolve the matrix material, and can be easily removed from the surface of the resulting powder. Specifically, an alcohol such as ethanol or isopropyl alcohol or the like may be mentioned. A cooling method of the cooling medium is not particularly limited, and cooling with dry ice or the like, cooling with an apparatus such as cooler, or the like can be freely selected depending on facilities.

The cooling temperature is a temperature sufficient for rapid cooling and solidification of the homogeneous mixture extruded from the die of the extruder and a temperature at which the resulting solid product is not softened. Specifically, the temperature may be about -10 to -30°C. The solid product obtained here is one solidified by rapid cooling of the matrix material comprising the polyphenol and the like locked therein. The pressure for extrusion varies depending on the kind of the raw material and the model of the extruder but may be a pressure at which the matrix material containing the polyphenol and the like locked therein is continuously extruded from the die of the extruder in a filamentous shape. Specifically, in the case where the extruder comprising a withstand pressure vessel fitted with an extrusion plate is used, after the extruder is tightly closed, it is suitable to impart about 5 to 50 psig of the pressure by a means such as introduction of nitrogen gas.

The solid product obtained by rapid cooling with extrusion of the homogeneous mixture is cut or pulverized by a means such as stirring to form a locked-in type powder. The resulting powder is separated into a powder and a cooling medium by a means such as centrifugation. In order to avoid re-caking of the powder, it is preferred to add a caking inhibitor. As the caking inhibitor, silicone dioxide, calcium tertiary sulfate, calcium tertiary phosphate, or the like can be used. The amount of the caking inhibitor to be added is suitably about 0.1 to 1% by weight based on the total weight.

Then, the powder is dried. The drying method is not particularly limited, but it is necessary to select a method which does not destroy the particle structure of the powder. Preferably, drying under reduced pressure by means of a vacuum rotary dryer or the like is preferred. The water content of the thus obtained powder is preferably about 6% by weight or less. Furthermore, if necessary, sieving or the like can be performed to obtain a locked-in type powder.

The average particle diameter of the locked-in type powder is not particularly limited but is usually a size of about 2 to 250 µm from the viewpoints of the ability for homogeneous mixing with a fragrance or cosmetic base, retention property of the deodorizing function and antioxidizing function of the polyphenol at use, touch at use, and the like.
The thus obtained locked-in type powder is blended into various fragrances or cosmetics which are a solid preparation including a powder pack, a whitening powder, a powder bath agent, a tablet bath agent, a bubble bath, a deodorant powder, a powder detergent, a detergent for an automatic dish washer, a powder bleach, a sliminess remover, a denture cleanser, and the like. The main purpose is to enhance a deodorizing effect at use of the fragrances or cosmetics but, in the case where a fragrance is coexisted with the polyphenols, there is a purpose of enhancing odor at use. Particularly, in the powder bath agent or tablet type bath agent, as a base agent, sodium sulfate is used, or in the case of an effervescent bath agent, sodium sulfate and sodium hydrogen carbonate are used. Moreover, in the detergent, in addition to a surfactant, sodium sulfate, sodium carbonate, sodium phosphate, or the like is used as a builder and an additive such as an enzyme is also used. In the case of the locked-in type powder comprising the polyphenols locked therein, since the polyphenols are not affected by such a base compound, a satisfactory deodorizing effect is exhibited even when used after long-term storage. Particularly, a fragrance or cosmetic, particularly a bath agent or a detergent, which comprises an alkali ingredient blended therein, retains a good deodorizing function. Therefore, as the fragrance or cosmetic, a bath agent and a detergent are mentioned as preferable examples.

The basic substance (alkali ingredient) which is an ingredient to be used in the invention is a known chemical substance and is not particularly limited so far as it can attain the intended purpose of the invention. Specific basic substance includes carbonate salts or hydrogen carbonate salts such as sodium carbonate, potassium carbonate, sodium hydrogen carbonate, potassium hydrogen carbonate, ammonium carbonate, and guanidine carbonate; borate salts such as potassium borate and sodium borate; silicate salts such as potassium silicate, sodium silicate No. 1, sodium silicate No. 2, sodium silicate No. 3, sodium orthosilicate, and sodium metasilicate; sodium monohydrogen phosphate, sodium sulfite, sodium hydroxide, calcium hydroxide, potassium hydroxide, magnesium hydroxide, ammonium hydroxide, sodium pyrophosphate, potassium pyrophosphate, sodium percarbonte, sodium perborate, potassium persulfate, and potassium hydrogen monopersulfate.
The blending amount of the basic substance is not particularly limited in the invention and an aqueous solution of the solid preparation comprising the locked-in type powder preferably shows pH of more than 7 and 13 or less, more preferably shows pH of 8 to 13.

Into the solid preparation of the invention, an enzyme may be further incorporated. The enzyme may be an enzyme usually used in the detergent, may be an enzyme having a deodorizing function such as a function of decomposing a malodor component, or may be an oxidase for a phenolic compound. These enzymes are known. Examples of the enzymes include carbohydrases, lipases, proteases, phytases, laccase, and the like.
Since the blending amount of the above enzyme varies depending on the kind and amount of the polyphenol used, the amount cannot be categorically defined and is not particularly limited so far as the intended purpose can be attained.

The surfactants include nonionic type (polyoxyethylene alkyl ether, fatty acid alkylolamide, etc.), acylglutamic acid type, and the like. The surfactants are preferably used each alone or as a combination of two or more of them. Examples of the polyoxyethylene alkyl ether include polyoxyethylene stearyl, polyoxyethylene hydrogenated castor oil, and the like. Examples of the fatty acid alkylolamide include coconut-oil fatty acid diethanolamide. The acylglutamic acid type includes glutamate esters of a saturated and unsaturated fatty acid having 12 to 18 carbon atoms, and a coconut-oil fatty acid, a hydrogenated coconut-oil fatty acid, a palm-oil fatty acid, a hydrogenated palm-oil fatty acid, a beef-tallow fatty acid, a hydrogenated beef-tallow fatty acid, and the like which are mixtures of the saturated and unsaturated fatty acid having 12 to 18 carbon atoms and, specific examples include N-coconut-oil fatty acid-acyl-L-glutamic triethanolamine, lauroyl-L-glutamic triethanolamine, sodium N-coconut-oil fatty acid-acyl-L-glutamate, sodium N-lauroyl-L-glutamate, sodium N-myristoyl-L-glutamate, sodium N-coconut-oil fatty acid-hydrogenated beaf-tallow fatty acid-acyl-L-glutamate, potassium N-coconut-oil fatty acid-acyl-L-glutamate, and the like.

In the case where the solid preparation for fragrance or cosmetic blending of the invention is a powder, capsule, or tablet type bath agent, the above locked-in type powder and fragrance and, if necessary, a moisturizing agent, an enzyme agent, a binder, a disrupting agent, a dispersant, a pigment, a surfactant, a buffer, a stabilizer, a pH regulator, a galenical component, and the like can be blended into a base material comprising sodium sulfate or sodium hydrogen carbonate as an effervescent agent.

Moreover, in the case where the solid preparation for fragrances or cosmetics of the invention is a powder or tablet type detergent, the above locked-in type powder and fragrance and, if necessary, a moisturizing agent, an enzyme agent, a disintegrator, a pigment, a buffer, an alkali agent, a bleach, a softening agent, a deposition inhibitor, a powderization agent, and the like can be blended into a surfactant and a builder.

In the solid preparation for fragrances or cosmetics of the invention, since the polyphenols are locked in the locked-in type capsule, the polyphenols are stably retained and, even after long-term storage, an excellent appearance of the fragrance or cosmetic can be retained without no coloration thereof. Furthermore, the deodorizing effect and the antioxidizing function are retained at use of the fragrance or cosmetic. Also, there is a characteristic that the influence of the base material used in the fragrance or cosmetic is hardly exerted. In the case where the fragrance or cosmetic is a bath agent or a detergent, a particularly excellent effect can be obtained. Moreover, when an alkali ingredient is blended into the fragrance or cosmetic, it can exhibit excellent deodorizing effect and antioxidizing function in conjunction with the polyphenols released from the locked-in type powder.
Furthermore, when a fragrance is used in the fragrance or cosmetic or solid preparation of the invention, since the fragrance is locked in the locked-in type capsule, the fragrance is hardly affected by the base material used in the fragrance or cosmetic and thereby a solid preparation for fragrances or cosmetics exhibiting no decrease in odor and no change in odor quality during the storage can be obtained.
For example, the following will describe a bath agent comprising a fragrance. Three types of bath agents were obtained by adding 1.0 part by weight of a locked-in type powder comprising a polyphenol of the invention, wherein each of catechin, gallic acid, and a coffee raw bean extract is locked in an amount of 0.1 part by weight, to 99.0 parts by weight of a bath agent base comprising 49.0 parts by weight of sodium sulfate, 49.0 parts by weight of sodium hydrogen carbonate, 1.0 part by weight of silicic anhydride, and 1.0 part by weight of a fragrance, followed by homogeneous mixing. When the three types of the bath agents were stored at 25°C or 50°C for one month, it was found that no change in odor quality was observed for all the three types of the bath agents. On the other hand, when three types of the bath agents prepared by blending 0.1 part by weight of each of catechin, gallic acid, and a coffee raw bean extract with 99.9 parts by weight of the above bath agent base were stored at 25°C or 50°C for one month, it was found that change in odor quality was observed for all the three types of the bath agents.

### (Examples)

The following will specifically describe the invention with reference to Examples, Comparative Examples, Test Examples, Production Examples and the like, but the invention is by no means limited to the following examples.

### Example 1: Production of locked-in type powder A

Together with 10 parts by weight of catechin (polyphenol powder GTP90: manufactured by Aiya Japan Corporation) dispersed in 27 mL of ion-exchange water, 66.79 parts by weight of isomalt (palatinit), 0.95 part by weight of a processed starch (PURITY GUM 59: manufactured by National Starch and Chemical Company), and 22.26 parts by weight of dextrin (Pinedex #1: manufactured by Matsuo Chemical Industry Co., Ltd.) were heated under stirring to obtain a homogeneous melt toffee. Then, the melt was extruded into an isopropyl alcohol cooled at -10°C in a filamentous shape using an extruder and rapidly cooled, followed by stirring and pulverizing. Subsequently, the pulverized product was centrifuged to remove isopropyl alcohol, then a homogeneous locked-in type powder A was obtained.

### Example 2: Production of locked-in type powder B

Together with 10 parts by weight of gallic acid (manufactured by Junsei Chemical Co., Ltd.) dispersed in 27 mL of ion-exchange water, 66.79 parts by weight of isomalt (palatinit), 0.95 part by weight of a processed starch (PURITY GUM 59: manufactured by National Starch and Chemical Company), and 22.26 parts by weight of dextrin (Pinedex #1: manufactured by Matsuo Chemical Industry Co., Ltd.) were heated under stirring to obtain a homogeneous melt. Then, the melt was extruded into an isopropyl alcohol cooled at -10°C in a filamentous shape using an extruder and rapidly cooled, followed by stirring and pulverizing. Subsequently, the product was centrifuged to remove isopropyl alcohol, then a homogeneous locked-in type powder B was obtained.

### Production Example 1: Preparation of coffee raw bean extract

After raw coffee beans were pulverized with a pulverizer (mesh: 5 mm), water was added thereto and extraction was performed at 85 to 95°C for 2 hours. After the extract was filtered, the filtrate was adsorbed on a XAD-2 (manufactured by Organo Corporation) column. After washing with water, an eluate eluted with methanol was concentrated to dry to obtain a coffee raw bean extract.

### Example 3: Production of locked-in type powder C

Together with 10 parts by weight of the coffee raw bean extract (Production Example 1) dispersed in 27 mL of ion-exchange water, 66.79 parts by weight of isomalt (palatinit), 0.95 part by weight of a processed starch (PURITY GUM 59: manufactured by National Starch and Chemical Company), and 22.26 parts by weight of dextrin (Pinedex #1: manufactured by Matsuo Chemical Industry Co., Ltd.) were heated under stirring to obtain a homogeneous melt. Then, the melt was extruded into an isopropyl alcohol cooled at -10°C in a filamentous shape using an extruder and rapidly cooled, followed by stirring and pulverizing. Subsequently, the product was centrifuged to remove isopropyl alcohol, then a homogeneous locked-in type powder C was obtained.

### Example 4: Production of dish-washer powder detergent (L)

To 99.0 parts by weight of a dish-washer powder detergent (CHARMY: manufactured by Lion Corporation) was added 1.0 part by weight of the locked-in type powder A produced in Example 1, followed by homogeneous mixing.

### Example 5: Production of dish-washer powder detergent (M)

To 99.0 parts by weight of a dish-washer powder detergent (CHARMY: manufactured by Lion Corporation) was added 1.0 part by weight of the locked-in type powder B produced in Example 2, followed by homogeneous mixing.

### Example 6: Production of dish-washer powder detergent (N)

To 99.0 parts by weight of a dish-washer powder detergent (CHARMY: manufactured by Lion Corporation) was added 1.0 part by weight of the locked-in type powder C produced in Example 3, followed by homogeneous mixing.

### Comparative Example 1: Production of dish-washer powder detergent (P)

To 99.9 parts by weight of a commercially available dish-washer powder detergent (CHARMY: manufactured by Lion Corporation) was added 0.1 part by weight of catechin (polyphenol powder GTP90: manufactured by Aiya Japan Corporation), followed by homogeneous mixing.

### Comparative Example 2: Production of dish-washer powder detergent (Q)

To 99.9 parts by weight of a dish-washer powder detergent (CHARMY: manufactured by Lion Corporation) was added 0.1 part by weight of gallic acid, followed by homogeneous mixing.

### Test Example 1

The dish-washer powder detergents (L) to (N), (P), (Q) immediately after production and they were stored in a state placed in individual containers at 5°C, 25°C, or 50°C for one month. Thereafter, appearance and odor of each of the dish-washer powder detergents (L) to (N), (P), and (Q) were evaluated in a sensory manner based on the following evaluation standard. The evaluation results were determined based on average points obtained from five panelists.
The results are shown in Table 2.

**[Table 1]**

| Composition of Dish-Washer Powder Detergent (part by weight) | | | | | |
|---|---|---|---|---|---|
| | Comparative Example 1 | Comparative Example 2 | Example 1 | Example 2 | Example 3 |
| Dish-washer powder detergent | 99.9 | 99.9 | 99.0 | 99.0 | 99.0 |
| Catechin | 0.1 | | | | |
| Gallic acid | | 0.1 | | | |
| Locked-in type powder A | | | 1.0 | | |
| Locked-in type powder B | | | | 1.0 | |
| Locked-in type powder B | | | | | 1.0 |

In the table, "1.0" in the powder of Example 1 includes a polyphenol content of 0.1 part by weight (the same applied in other Examples).

### Evaluation standard (compared with detergent stored at 5°C)

Appearance
A No coloration (change in color) is observed [no change]
B Very slight coloration (change in color) is observed
C Slight coloration (change in color) is observed
D Considerable coloration (change in color) is observed
E Complete coloration (change in color) is observed
Odor
A No change in odor is observed [no change]
B Very slight change in odor is observed
C Slight change in odor is observed
D Considerable change in odor is observed
E Complete change in odor is observed

**[Table 2]**

| Evaluation of Dish-Washer Powder Detergent | | | | | | | | | | |
|---|---|---|---|---|---|---|---|---|---|---|
| | Comparative Example 1 | | Comparative Example 2 | | Example 4 | | Example 5 | | Example 6 | |
| | 25°C | 50°C | 25°C | 50°C | 25°C | 50°C | 25°C | 50°C | 25°C | 50°C |
| Appearance | C | C | A | B | A | A | A | A | A | A |
| Odor | C | C | C | C | A | A | A | A | A | A |

From Table 2, with regard to the dish-washer powder detergents produced by conventional methods, after storage at 50°C for one month, it is found that the degree of coloration (change in color) is high and appearance becomes extremely worse as well as the odor is changed and unbalanced. To the contrary, in the dish-washer powder detergents blended into the locked-in type powders of the invention, even after storage at 50°C for one month, it is found that appearance and odor are retained as same as the appearance and odor before storage.

### Comparative Example 3: Production of powder detergent (P) for clothing

To 99.9 parts by weight of a powder detergent for clothing (Heyabosi TOP: manufactured by Lion Corporation) was added 0.1 part by weight of catechin (polyphenol powder GTP90: manufactured by Aiya Japan Corporation), followed by homogeneous mixing.

### Comparative Example 4: Production of powder detergent (Q) for clothing

To 99.9 parts by weight of a powder detergent for clothing (Heyaboshi TOP: manufactured by Lion Corporation) was added 0.1 part by weight of gallic acid, followed by homogeneous mixing.

### Comparative Example 5: Production of powder detergent (R) for clothing

To 99.9 parts by weight of a powder detergent for clothing (Heyaboshi TOP: manufactured by Lion Corporation) was added 0.1 part by weight of the coffee raw bean extract of Production Example 1, followed by homogeneous mixing.

### Example 7: Production of powder detergent (L) for clothing

To 99.0 parts by weight of a commercially available powder detergent for clothing (Heyaboshi TOP: manufactured by Lion Corporation) was added 1.0 part by weight of the locked-in type powder A produced in Example 1, followed by homogeneous mixing.

### Example 8: Production of powder detergent (M) for clothing

To 99.0 parts by weight of a commercially available powder detergent for clothing (Heyaboshi TOP: manufactured by Lion Corporation) was added 1.0 part by weight of the locked-in type powder B produced in Example 2, followed by homogeneous mixing.

### Example 9: Production of powder detergent (N) for clothing

To 99.0 parts by weight of a commercially available powder detergent for clothing (Heyaboshi TOP: manufactured by Lion Corporation) was added 1.0 part by weight of the locked-in type powder C produced in Example 3, followed by homogeneous mixing.

**[Table 3]**

| Composition of Powder Detergent for Clothing (part by weight) | | | | | | |
|---|---|---|---|---|---|---|
| | Comparative Example 3 | Comparative Example 4 | Comparative Example 5 | Example 7 | Example 8 | Example 9 |
| powder detergent for clothing | 99.9 | 99.9 | 99.9 | 99.0 | 99.0 | 99.0 |
| Catechin | 0.1 | | | | | |
| Gallic acid | | 0.1 | | | | |
| Coffee raw bean extract | | | 0.1 | | | |
| Locked-in type powder A | | | | 1.0 | | |
| Locked-in type powder B | | | | | 1.0 | |
| Locked-in type powder B | | | | | | 1.0 |

### Test Example 2

The powder detergents (P) to (R), (L) to (N) for clothing immediately after production and they were stored in a state placed in individual containers at 5°C, 25°C, or 50°C for one month. Thereafter, appearance and odor of each of the powder detergents (P) to (R) and (L) to (N) for clothing were evaluated in the same manner as in Test Example 1.
The results are shown in Table 4.

**[Table 4]**

| Evaluation of Powder Detergent for Clothing | | | | | | | | | | | | |
|---|---|---|---|---|---|---|---|---|---|---|---|---|
| | Comparative Example 3 | | Comparative Example 4 | | Comparative Example 5 | | Example 7 | | Example 8 | | Example 9 | |
| | 25°C | 50°C | 25°C | 50°C | 25°C | 50°C | 25°C | 50°C | 25°C | 50°C | 25°C | 50°C |
| Appearance | B | E | B | C | A | C | A | A | A | A | A | A |
| Odor | B | C | B | C | A | D | A | A | A | A | A | A |

From Table 4, with regard to the powder detergents for clothing produced by conventional methods, after storage at 50°C for one month, it is found that the degree of coloration (change in color) is high and appearance becomes extremely worse as well as the odor is changed and unbalanced. To the contrary, in the clothing powder detergents for clothing blended into the locked-in type powders of the invention, even after storage at 50°C for one month, it is found that appearance and odor are retained as same as the appearance and odor before storage.

While the invention has been described in detail and with reference to specific embodiments thereof, it will be apparent to one skilled in the art that various changes and modifications can be made therein without departing from the spirit and scope thereof.
The present application is based on Japanese Patent Application No. 2006-079796 filed on March 22, 2006, and the contents are incorporated herein by reference.
Also, all the references cited herein are incorporated as a whole.

### Industrial Applicability

In the solid preparation for fragrances or cosmetics of the invention, since the polyphenols are locked in a locked-in type capsule, the polyphenols are stably retained and, even after long-term storage, the fragrance or cosmetic is not colored and an excellent appearance can be retained. Furthermore, the deodorizing effect and the antioxidizing function are retained at use of the fragrance or cosmetic. Also, there is a characteristic that the influence of the base material used in the fragrance or cosmetic is hardly exerted. In the case where the fragrance or cosmetic is a bath agent or a detergent, a particularly excellent effect can be obtained. Moreover, when an alkali ingredient is blended in the fragrance or cosmetic, it can exhibit excellent deodorizing effect and antioxidizing function in conjunction with the polyphenols released from the locked-in type powder.
Furthermore, when a fragrance is used in the fragrance or cosmetic or solid preparation of the invention, the fragrance is locked in the locked-in type capsule, the fragrance is hardly affected by the base material used in the fragrance or cosmetic and thereby a solid preparation for fragrances or cosmetics exhibiting no decrease in odor and no change in odor quality during the storage can be obtained.

## Claims

1. A locked-in type powder comprising a polyphenols.

2. The locked-in type powder according to claim 1, wherein a content of the polyphenols is 0.2 to 20% by weight based on the total weight of the locked-in type powder.

3. The locked-in type powder according to claim 1 or 2, wherein the polyphenols is locked in a matrix material.

4. The locked-in type powder according to any one of claims 1 to 3, wherein the polyphenols is a polyphenol having a hydroquinone or an o-diphenol structure.

5. The locked-in type powder according to any one of claims 1 to 4, which further comprises at least one of a flavor and a fragrance.

6. A solid preparation comprising the locked-in type powder according to any one of claims 1 to 5.

7. The solid preparation according to claim 6, wherein the solid preparation is a fragrance or cosmetic.

8. The solid preparation according to claim 7, wherein the fragrance or cosmetic is a bath agent or a detergent.

9. The solid preparation according to any one of claims 6 to 8, which further comprises a basic ingredient.

10. The solid preparation according to any one of claims 6 to 8, which further comprises an enzyme.
